(19) Europäisches Patentamt
European Patent Office
Office européen des brevets

(11) Veröffentlichungsnummer: **0 164 643**
**A2**

(12) **EUROPÄISCHE PATENTANMELDUNG**

(21) Anmeldenummer: 85106561.5

(22) Anmeldetag: 29.05.85

(51) Int. Cl.[4]: C 11 C 3/04
C 07 C 67/03, C 07 C 69/003

(30) Priorität: 07.06.84 DE 3421217

(43) Veröffentlichungstag der Anmeldung: 18.12.85 Patentblatt 85/51

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Billenstein, Siegfried, Dr.
Samerbergweg 8
D-8269 Burgkirchen(DE)

(72) Erfinder: Kukla, Bruno
Kalkweg 11
D-8269 Burgkirchen(DE)

(72) Erfinder: Stühler, Herbert, Dr.
Hochfellnstrasse 12
D-8269 Burgkirchen(DE)

(54) **Verfahren zur Herstellung von Fettsäureestern kurzkettiger Alkohole.**

(57) Es wird ein Verfahren zur Herstellung von Fettsäureestern kurzkettiger primärer und sekundärer Alkohole mit 1 bis 5 C- Atomen durch Umesterung von Glyceriden mit den genannten kurzkettigen Alkoholen beschrieben. Bei diesem Verfahren wird durch das flüssige Glycerid bei Temperaturen von mindestens 210°C ein Strom des gasförmigen Alkohols hindurchgeleitet und mit diesem Strom das Produktgemisch aus Glycerin und Fettsäurealkylester aus der Reaktionszone ausgetragen, das anschließend einer Phasentrennung unterworfen wird.

EP 0 164 643 A2

Croydon Printing Company Ltd.

HOECHST AKTIENGESELLSCHAFT HOE 84/F 904 Dr.SC-ba

Verfahren zur Herstellung von Fettsäureestern kurzkettiger Alkohole

Die Erfindung betrifft ein Verfahren zur Herstellung von Fettsäureestern durch Umesterung von Glyceriden mit kurzkettigen Alkoholen.
Fettsäureester kurzkettiger Alkohole besitzen als Zwischenprodukte, beispielsweise für die Herstellung von Fettalkoholen oder Fettnitrilen oder bei der Herstellung von Seifen, erhebliche technische Bedeutung. Als Komponenten für gewisse Motorentreibstoffe, insbesondere Dieseltreibstoffe, können sie auch direkt Verwendung finden.

Herstellungsverfahren für Fettsäureester kurzkettiger Alkohole, ausgehend von Fetten und Ölen nativen Ursprungs, sind seit langem bekannt. In den US-Patentschriften 2 271 619 und 2 360 844 aus dem Jahr 1939 ist das grundlegende Verfahren beschrieben: Das Fett oder Öl wird mit dem kurzkettigen aliphatischen Alkohol und einem alkalischen Katalysator vermischt und auf etwa 80 °C erhitzt. Nach einer kurzen Zeitspanne beginnt sich das Reaktionsgut in zwei Schichten zu trennen, das Glycerin scheidet sich am Boden des Gefäßes ab und kann von dort abgetrennt werden. Überschüssiger Alkohol wird destillativ entfernt und der gebildete Fettsäureester dann destilliert oder gegebenenfalls in Fraktionen zerlegt. Dieses Verfahren ist seither in vielfacher Weise verbessert worden. Einen Überblick über den derzeitigen Stand gibt der Aufsatz in JAOCS, 61 (1984), Seite 343 ff., insbesondere Seiten 344 bis 346, auch unter Berücksichtigung der heute üblichen kontinuierlichen Verfahrensweise. Dort ist auch der wesentliche Nachteil dieses Umesterungsverfahrens angesprochen.
Soll die Reaktion bei milden Reaktionsbedingungen ablaufen

(bei 50 bis 70 °C und etwa Atmosphärendruck), so ist es zwingend erforderlich, die im Ausgangsprodukt enthaltenen freien Fettsäuren durch Vorveresterung oder andere Maßnahmen zu entfernen. Lediglich bei Führung des Prozesses unter hohem Druck und hoher Temperatur, beispielsweise bei 90 bar und 240 °C, und bei hohem Überschuß an Methanol kann auf diese vorherige Entfernung freier Fettsäuren verzichtet werden, so daß hier auch nicht entsäuerte Fette und Öle zum Einsatz kommen können.

Der Grund für diese Schwierigkeit liegt wahrscheinlich darin, daß die aus freien Fettsäuren mit dem alkalischen Katalysator sich bildenden Seifen auf das Glycerin emulgierend wirken und so dessen Abtrennung vom gebildeten Fettsäureester behindern oder unmöglich machen. Da jedoch die Abscheidung des Glycerins als separate Phase diesen Reaktionspartner aus dem Gleichgewicht entfernt und somit den Fortgang der Reaktion fördert, ist dieser Vorgang der unvollständigen Trennung oder Emulgierung sowohl wegen der Behinderung des Fortschreitens der Reaktion als auch wegen der dabei eintretenden Verunreinigung des Glycerins in hohem Grade unerwünscht.

Es sind schon eine ganze Reihe von Verfahrensänderungen und Verfahrensverbesserungen entwickelt worden, mit deren Hilfe diese Nachteile behoben werden sollen. So beschreibt die US-PS 3 383 614 ein Verfahren zur kontinuierlichen Alkoholyse von Fetten, wobei zunächst - gegebenenfalls in mehreren Schritten - eine Teilveresterung des Fettes oder Öls vorgenommen wird und entsprechend auch die Abscheidung des Glycerins in mehreren Stufen erfolgt. Gemäß US-PS 2 383 580 soll nach beendeter Reaktion zunächst der Katalysator inhibiert werden, indem die Reaktionsmischung neutralisiert wird, sodann wird der überschüssige Alkohol destillativ entfernt

und schließlich wird das verbleibende Gemisch im Vakuum destilliert, wobei sich das Kondensat rasch in eine Glycerin- und eine Fettsäurealkylester-Schicht trennt. Gemäß der US-PS 2 383 633 soll zunächst der überschüssige Alkohol abdestilliert und sodann die Trennung des verbleibenden Gemischs in Glycerin und Fettsäurealkylester durch Ansäuern mit Mineralsäure erleichtert werden. Insbesondere unter dem Gesichtspunkt einer einfachen Reaktionsführung und der Gewinnung des Glycerins in möglichst hoher Ausbeute und Reinheit sind alle diese Verfahren unbefriedigend.

Noch in jüngster Zeit wurde daher gemäß der US-PS 4 164 506 ein Verfahren zur Veresterung von nicht vorraffinierten Fetten dahingehend entwickelt, daß in einem Zweistufenverfahren zunächst in Gegenwart saurer Katalysatoren die freien Fettsäuren mit kurzkettigen Alkoholen in deren Ester umgewandelt werden und sodann die Umsetzung der Glyceride in die Fettalkylester in Gegenwart von Alkali unter Abscheidung von Glycerin vorgenommen wird.

Gemäß einer Veröffentlichung von J. Poré und J. Verstraete, Oléagineux 7 (1952) Nr. 11, Seite 641 bis 644, wurde auch bereits versucht, dieses Hindernis dadurch zu umgehen, daß ein saurer Katalysator verwendet und das Methanol dampfförmig zugeführt wird. Glycerin wird dabei meist nicht abgeschieden. Wird eine stufenweise Abscheidung des Glycerins aus dem Reaktionsgefäß durchgeführt, so soll sich die Ausbeute an Ester etwas erhöhen, es treten dann aber die gleichen Schwierigkeiten auf wie bei dem oben geschilderten Verfahren.

Somit besteht ein Bedürfnis nach einem Verfahren, das vor allem beim Einsatz nicht vorbehandelter Fette und

Öle, die freie Fettsäuren und/oder Schleimstoffe in größeren Mengen enthalten, nicht nachteilig beeinflußt wird. Dabei soll die von den Anlagekosten her sehr aufwendige Fahrweise unter hohem Druck vermieden werden und dennoch ohne aufwendige Vor- und Nachbehandlung Fettsäurealkylester und Glycerin in guter Qualität gewonnen werden.

Diesem Bedürfnis wird Rechnung getragen durch ein Verfahren zur Herstellung von Fettsäureestern kurzkettiger primärer und sekundärer Alkohole mit 1 bis 5 C-Atomen durch Umesterung von Glyceriden mit solchen kurzkettigen Alkoholen in Gegenwart von Umesterungskatalysatoren bei erhöhten Temperaturen, dadurch gekennzeichnet, daß das flüssige Glycerid bei Temperaturen von mindestens 210 °C mit einem Strom von gasförmigem Alkohol in innigen Kontakt gebracht wird, wobei dieser Strom in seiner Durchsatzmenge, bezogen auf die Zeiteinheit, mindestens so bemessen ist, daß er imstande ist, das gebildete Produktgemisch aus Glycerin und Fettsäureester gemeinsam rasch aus der Reaktionszone auszutragen, worauf das Produktgemisch nach Kondensation einer Phasentrennung in eine Fettsäureester- und in eine Glycerin-Phase unterworfen und der überschüssige gasförmige Alkohol in die Reaktionszone zurückgeführt wird.

Ausgangsstoffe für das erfindungsgemäße Verfahren sind Mono-, Di- und Triglyceride der allgemeinen Formel

$$\begin{array}{l} CH_2-O-X \\ | \\ CH\ -O-Y \\ | \\ CH_2-O-COR^3, \end{array}$$

worin X = $COR^1$ oder H, Y = $COR^2$ oder H ist und $R^1$, $R^2$ und $R^3$, die gleich oder verschieden sein können, aliphatische Kohlenwasserstoff-Gruppen mit 3 bis 23 C-Atomen bedeuten, wobei diese Gruppen gegebenenfalls mit

einer OH-Gruppe substituiert sein können oder beliebige Gemische aus solchen Glyceriden.

Das heißt, in dieser Formel können ein oder zwei Fettsäureester durch Wasserstoff ersetzt sein und die Fettsäureester $R^1CO-$, $R^2-CO-$ und $R^3CO-$ leiten sich ab von Fettsäuren mit 3 bis 23 Kohlenstoffatomen in der Alkylkette. $R^1$ und $R^2$ oder $R^1$, $R^2$ und $R^3$ können in der obengenannten Formel gleich oder verschieden sein, wenn es sich um Di- oder Triglyceride handelt. Die Reste $R^1$, $R^2$ und $R^3$ gehören den folgenden Gruppen an:

a) Alkylradikale, die verzweigt sein können, jedoch vorzugsweise geradkettig sind und 3 bis 23, vorzugsweise 7 bis 23 C-Atome haben;

b) olefinisch ungesättigte aliphatische Kohlenwasserstoffreste, die verzweigt sein können, jedoch vorzugsweise geradkettig sind, und die 3 bis 23, vorzugsweise 11 bis 21 und insbesondere 15 bis 21 C-Atome besitzen und die 1 bis 6, vorzugsweise 1 bis 3 Doppelbindungen enthalten, welche konjugiert oder isoliert sein können;

c) monohydroxy-substituierte Reste des Typs a) und b), vorzugsweise olefinisch ungesättigte Olefinreste, die 1 bis 3 Doppelbindungen besitzen, und insbesondere den Rest der Ricinolsäure.

Die Acylradikale $R^1CO-$, $R^2CO-$ und $R^3CO-$ solcher Glyceride, die als Ausgangsmaterialien für das Verfahren der vorliegenden Erfindung geeignet sind, leiten sich ab von den folgenden Gruppen aliphatischer Carbonsäuren (Fettsäuren):

a) Alkansäuren oder deren alkylverzweigte, insbesondere methylverzweigte Derivate, die 4 bis 24 Kohlenstoffatome besitzen, wie zum Beispiel Buttersäure, Valeriansäure, Capronsäure, Heptansäure, Caprylsäure, Pelargonsäure, Caprinsäure, Undecansäure, Laurinsäure,

Tridecansäure, Myristinsäure, Pentadecansäure, Palmitinsäure, Margarinesäure, Stearinsäure, Nonadecansäure, Arachidinsäure, Behensäure, Lignocerinsäure, 2-Methylbutansäure, Isobuttersäure, Isovaleriansäure, Pivalinsäure, Isocapronsäure, 2-Ethylcapronsäure, die stellungsisomeren Methylcaprinsäuren, Methyllaurinsäuren und Methylstearinsäuren, 12-Hexylstearinsäure, Isostearinsäure oder 3,3-Dimethylstearinsäure.

b) Alkensäuren, Alkadiensäuren, Alkatriensäuren, Alkatetraensäuren, Alkapentaensäuren und Alkahexaensäuren sowie deren alkylverzweigte, speziell methylverzweigte Derivate mit 4 bis 24 C-Atomen, wie zum Beispiel Crotonsäure, Isocrotonsäure, Caproleinsäure, 3-Lauroleinsäure, Myristoleinsäure; Palmitoleinsäure, Ölsäure, Elaidinsäure, Erucasäure, Brassidinsäure, 2,4-Decadiensäure, Linolsäure, 11,14-Eicosadiensäure, Eleostearinsäure, Linolensäure, Pseudoeleostearinsäure, Arachidonsäure, 4,8,12,15,18,21-Tetracosahexaensäure oder trans-2-Methyl-2-butensäure.

$c_1$) Monohydroxyalkansäuren mit 4 bis 24 C-Atomen, vorzugsweise mit 12 bis 24 C-Atomen, vorzugsweise unverzweigt, wie zum Beispiel Hydroxybuttersäure, Hydroxyvaleriansäure, Hydroxycapronsäure, 2-Hydroxydodecansäure, 2-Hydroxytetradecansäure, 15-Hydroxypentadecansäure, 16-Hydroxyhexadecansäure, Hydroxyoctadecansäure.

$c_2$) Ferner Monohydroxyalkensäuren mit 4 bis 24, vorzugsweise mit 12 bis 22 und insbesondere mit 16 bis 22 C-Atomen (vorzugsweise unverzweigt) und mit 1 bis 6, vorzugsweise mit 1 bis 3 und insbesondere mit einer ethylenischen Doppelbindung, wie zum Beispiel Ricinoleinsäure oder Ricinelaidinsäure.

Bevorzugte Ausgangsstoffe für das erfindungsgemäße Verfahren sind vor allem die natürlichen Fette, die Gemische aus überwiegend Triglyceriden und kleinen Anteilen aus Diglyceriden und/oder Monoglyceriden darstellen, wobei auch diese Glyceride meist wiederum Gemische darstellen und verschiedenartige Fettsäurereste im obengenannten Bereich, insbesondere solche mit 8 und mehr C-Atomen, enthalten. Beispielsweise seien genannt pflanzliche Fette, wie Olivenöl, Kokosfett, Palmkernfett, Babussuöl, Palmöl, Erdnußöl, Rüböl, Ricinusöl, Sesamöl, Cottonöl, Sonnenblumenöl, Sojaöl, Hanföl, Mohnöl, Avocadoöl, Baumwollsaatöl, Weizenkeimöl, Maiskeimöl, Kürbiskernöl, Traubenkernöl, Kakaobutter oder auch Pflanzentalge, ferner tierische Fette, wie Rindertalg, Schweinefett, Knochenfett, Hammeltalg, Japantalg, Walöl und andere Fischöle sowie Lebertran. Ebenso eingesetzt werden können aber auch einheitliche Tri-, Di- und Monoglyceride, sei es, daß diese aus natürlichen Fetten isoliert oder auf synthetischem Wege gewonnen wurden. Hier seien beispielsweise genannt: Tributyrin, Tricapronin, Tricaprylin, Tricaprinin, Trilaurin, Trimyristin, Tripalmitin, Tristearin, Triolein, Trielaidin, Trilinoliin, Trilinolenin, Monopalmitin, Monostearin, Monoolein, Monocaprinin, Monolaurin, Monomyristin oder gemischte Glyceride, wie beispielsweise Palmitodistearin, Distearoolein, Dipalmitoolein oder Myristopalmitostearin.

Von wesentlicher Bedeutung für das erfindungsgemäße Verfahren ist es, das Glycerin bei seiner Freisetzung durch die Umesterungsreaktion rasch aus der Reaktionszone zu entfernen. Dies wird erreicht dadurch, daß Glycerin gemeinsam mit dem gebildeten Fettsäureester in einem Strom von gasförmigem Alkanol ausgetragen wird. Dabei ist dieser Strom so zu bemessen, daß der

Austrag in das Kondensationsgefäß möglich ist. Dazu bedarf es einer gewissen Mindest-Durchsatzmenge an dampfförmigem, kurzkettigem Alkohol, bezogen auf die Menge an Glycerid, multipliziert mit der Zeit, in der der Alkohol durchgeleitet wird. Diese Durchsatzmenge wird hier angegeben in mol Alkohol/kg Glycerid mal Stunde. Als Menge an Glycerid wird dabei bei einer chargenweisen Verfahrensweise die vorgelegte Ausgangsmenge an Glycerid verstanden. Demgemäß wird der Rahmen dieser Erfindung nicht verlassen, wenn im weiteren Verlauf der Reaktion, insbesondere gegen deren Ende, diese Mindest-Durchsatzmenge gegebenenfalls auch unterschritten wird, entsprechend der noch im Reaktionsgefäß befindlichen verminderten Menge an Glycerid bzw. Fett. Bei einem Kontinuum richtet sich die Durchsatzmenge von gasförmigem Alkohol nach der im Reaktionsraum vorgelegten und/oder durch Zufuhr aufrechterhaltenen Menge an Glycerid. Die genannte Mindest-Durchsatzmenge liegt bei 8 mol Alkohol/kg Glycerid mal Stunde. Im allgemeinen wird man diese Durchsatzmenge aber oberhalb dieses Wertes wählen, was insbesondere von der Art des eingesetzten Glycerids, Fetts oder Öls abhängt, aber auch von den apparativen Gegebenheiten in der Zone zwischen Reaktions- und Kondensationsgefäß, wo eine vorzeitige Kondensation des ausgetragenen Produktgemischs verhindert werden soll. Außerdem hängt die erforderliche Durchsatzmenge noch ab von der Kettenlänge des eingesetzten Alkohols bzw. im Zusammenhang damit, von der Flüchtigkeit des gebildeten Esters sowie auch von der Reaktionstemperatur innerhalb des erfindungsgemäßen Temperaturbereichs. Vorzugsweise liegt die Durchsatzmenge, bezogen auf Kilogramm Glycerid mal Stunde, im Bereich von 20 bis 40 mol Alkohol. Dabei ist die Obergrenze nicht kritisch, sondern allenfalls durch wirtschaftliche Überlegungen bedingt

wenn die Menge des Kreisgases nicht unnötig groß werden soll. Es können mit Vorteil zu dieser Durchsatzmenge an Alkohol bis zu 30 %, vorzugsweise bis zu 15 %, an Inertgas, wie beispielsweise Stickstoff, hinzugefügt werden.

Als kurzkettige Alkohole für die Veresterungsreaktion kommen primäre und sekundäre Alkohole mit 1 bis 5 C-Atomen in gerader oder verzweigter Kette in Betracht, also beispielsweise Pentanol, Butanol, Isobutanol, bevorzugt aber Ethanol, Propanol, Isopropanol und insbesondere Methanol.

Die Reaktionstemperatur wird im Bereich von 210 bis 280 °C, vorzugsweise von 230 bis 260 °C, gewählt. Die Wahl hängt vor allem von der Flüchtigkeit des jeweils gebildeten Fettsäurealkylesters und auch von der Durchsatzmenge an gasförmigem Alkohol ab. Die Temperatur kann innerhalb dieses Bereichs während der Reaktion über den Anfangswert ansteigen oder unter den Anfangswert absinken, gegebenenfalls auch kontinuierlich oder nach einem festgelegten Temperaturprogramm verändert werden; vorzugsweise wird die Reaktionstemperatur jedoch bis zum Reaktionsende beibehalten.

Die Reaktion wird üblicherweise bei Atmosphärendruck durchgeführt, jedoch wird auch bei einer Anwendung von Unter- oder Überdruck der Rahmen dieser Erfindung nicht verlassen, insbesondere wenn dieser sich aus den im Kreislauf herrschenden Druckverhältnissen ergibt.

Für die Herstellung von Fettsäurealkylestern und Glycerin aus Glyceriden und kurzkettigen Alkoholen ist die Gegenwart eines üblichen Umesterungskatalysators erforderlich, wobei bekannte Katalysatoren eingesetzt werden.

die auch bisher bei der Alkoholyse von Fetten bereits Verwendung gefunden haben. Solche für das erfindungsgemäße Verfahren geeigneten Umesterungskatalysatoren sind beispielsweise Alkalimetallsalze und andere basische Verbindungen von Alkalimetallen mit Salzcharakter, wie unter anderem Alkalicarbonate und -hydrogencarbonate, Alkalistearate, -laurate, -oleate, -palmitate (oder Gemische solcher Seifen), Alkalisalze von anderen Carbonsäuren wie Alkaliacetate, Alkalioxide, -hydroxide, -alkoholate, -hydride oder auch Alkaliamide. Der Ausdruck Alkalimetalle umfaßt hier alle Metalle der ersten Hauptgruppe, wobei aus wirtschaftlichen Gründen Natrium und Kalium bevorzugt sind.
Weitere Gruppen von geeigneten Umesterungskatalysatoren stellen die Schwermetallseifen dar, also Fettsäuresalze beispielsweise des Mangans, Zinks, Cadmiums und des zweiwertigen Bleis; ferner Schwermetallsalze von Alkylbenzolsulfonsäuren, Alkansulfonsäuren und Olefinsulfonsäuren, wie insbesondere die Salze des Zinks, Titans, Bleis, Chroms, Kobalts und Cadmiums. Schließlich hat sich auch Antimontrioxid als geeignet erwiesen. Die erforderliche Menge an Umesterungskatalysator kann beim erfindungsgemäßen Verfahren in ziemlich weiten Grenzen schwanken, insbesondere je nach Verunreinigung des eingesetzten Fetts. Sie liegt im Bereich von 0,05 bis 5 Gew.-%, vorzugsweise von 0,2 bis 3 Gew.-%, bezogen auf eingesetztes Glycerid. Bei einer vollkontinuierlichen Reaktionsweise bezieht sich diese Menge auf die vorgelegte Menge an Glycerid (die gegebenenfalls ihrerseits in einem separaten Kreis geführt werden kann) mit der Maßgabe, daß die konintuierlich nachgeführte Menge dem Austrag der bei der Umesterung gebildeten Produkte, jeweils pro Zeiteinheit, entspricht.

Die Durchführung des erfindungsgemäßen Verfahrens geht beispielsweise wie folgt vonstatten: In ein übliches Rührwerksgefäß, das mit Temperaturanzeige, Heizvorrichtung und einer geeigneten Vorrichtung zur Einleitung des dampfförmigen Alkoholstroms und gegebenenfalls des Inertgases in das flüssige Reaktionsgut ausgestattet ist, wird das Glycerid, also üblicherweise ein natürliches Fett oder Öl, vorgelegt, mit dem Katalysator versetzt und das vorgelegte Fett auf die Reaktionstemperatur erwärmt. Sobald diese erreicht ist, wird über die Einleitungsvorrichtung der Alkoholstrom gasförmig eingeleitet, wobei für eine gute Gas-Flüssigkeitsdurchmischung Sorge zu tragen ist. Vom Reaktionsgefäß wird der Alkoholstrom zusammen mit dem ausgetragenen Produktgemisch in ein Kondensationsgefäß oder ein System von Kondensationsgefäßen geleitet, wobei für einen möglichst kurzen und gut isolierten Übergang zu sorgen ist, um einen Rücklauf in das Reaktionsgefäß zu vermeiden. Die Temperatur im Kondensationssystem soll dabei etwa 10 bis 60 °C, vorzugsweise 20 bis 40 °C, über dem Siedepunkt des jeweiligen Alkohols liegen, mit der Maßgabe, daß bei Alkoholen mit 4 bis 5 C-Atomen der Abstand oberhalb des Siedepunkts nicht größer als 40 °C sein soll. Während so der Alkohol das Kondensationsgefäß gasförmig passiert und - gegebenenfalls nach einer Wäsche - im Kreislauf in die Reaktionszone zurückgeführt wird, unterwirft man das Produktgemisch aus dem gebildeten Fettsäurealkylester und Glycerin einer Phasentrennung. Wenn gegebenenfalls mehrere Kondensationsgefäße vorgeschaltet werden, erfolgt die Phasentrennung dann nach Vereinigung aller Kondensate. Die Kondensation kann beispielsweise in einem oder auch in mehreren hintereinandergeschalteten Wärmetauschern oder auch durch Umwälzen des Kondensats über Kühler und Füllkörper-, Boden- oder Sprühkolonnen

bewerkstelligt werden. Die Phasentrennung erfolgt zum Beispiel in Absetzgefäßen oder in Zentrifugen. Nach erfolgter Phasentrennung wird das Glycerin der Aufarbeitung zugeführt. Der überschüssige Alkohol wird nach Verdichtung über den Kreislauf wieder in das Reaktionsgefäß geführt. Insbesondere bei Fetten mit höheren Anteilen an Fettsäuren kann es zweckmäßig sein, einen Teil des Alkohols zur Herabsetzung des Gehalts an Reaktionswasser von Zeit zu Zeit aus dem Kreislauf auszuschleusen und durch frischen Alkohol zu ersetzen.

Das erfindungsgemäße Verfahren kann auch vollkontinuierlich durchgeführt werden, beispielsweise dadurch, daß in einer Rieselbettsäule oder Reaktionskolonne im Gegenstrom Methanol von unten und aufgeheiztes flüssiges Fett von oben zugeführt wird, wobei dann am Kopf der Kolonne die flüchtigen Anteile gemeinsam ausgetragen werden und das Methanol, wie oben beschrieben, im Kreis geführt und die Produkte der Phasentrennung unterworfen und aufgearbeitet werden. Ebenso können auch in einer Blasensäule Alkohol und das Glycerid in guter Durchmischung von unten nach oben geführt werden. Besonders zweckmäßig in bezug auf eine gute Durchmischung von Glycerid und Alkohol ist die Reaktionsführung im sogenannten Schleifenreaktor. Bei einer solchen Reaktionsweise wird auch der Fettanteil und der darin befindliche Katalysator in einem (separaten) Kreis geführt, wobei der umgesetzte Anteil an Ausgangs-Glycerid in diesen Kreis hinein ersetzt wird. Bei der kontinuierlichen Durchführung des erfindungsgemäßen Verfahrens wird das Glycerid im Reaktor vorgelegt. Nach Beginn der Umsetzung wird Glycerid in dem Maße ergänzt, wie es bei der Umsetzung verbraucht, das heißt in Form der Reaktionsprodukte abgeführt wird.

Das erfindungsgemäße Verfahren bringt eine Reihe von erheblichen Vorteilen gegenüber den bisher gebräuchlichen Verfahren mit sich:

1.) Das Verfahren kann sowohl mit vorher raffinierten als auch mit unraffinierten Fetten und Ölen durchgeführt werden. Das bedeutet nicht nur, daß die zeitraubende und kostspielige Entfernung der Fettsäuren entfällt - sei es in einem separaten Verfahren, oder sei es in Form irgendwelcher vorgeschalteter Reaktionen innerhalb des eigentlichen Verfahrensganges -, sondern auch, daß sogenannte nicht entschleimte Fette (beispielsweise unfiltriertes Tierkörperfett) unmittelbar eingesetzt werden können. Deren Einsatz bei Verfahren, die unter Absetzen des Glycerins vonstatten gehen, ist problematisch, da die im Fett enthaltenen Schleimstoffe, die als natürliche Emulgatoren wirken, gleichfalls stabile Emulsionen begünstigen und somit die Abscheidung des Glycerins behindern.

2.) Das erfindungsgemäße Verfahren benötigt nicht die Anwendung von hohem Druck, wie er bei den gebräuchlichen kontinuierlichen Absetzverfahren angewandt wird. Es kann bei Normaldruck gearbeitet werden, oder es sind höchstens geringe Überdrücke (bis 5 bar) erforderlich, die durch die Verhältnisse im Reaktionskreislauf bedingt sind.

3.) Die Überführung des gebildeten Esters im Methanolstrom liefert diesen in so hoher Reinheit, daß eine destillative Nachreinigung praktisch entbehrlich wird. Während bei den bekannten Verfahren Rohglycerin in guter Ausbeute und guter Qualität nur aus hochraffinierten Fetten und nur nach Abtrennung des Katalysators erhalten wird, gelingt dies beim erfindungsgemäßen Verfahren auch aus Fetten minderer Qualität.

Die erfindungsgemäß erhaltenen Fettsäureester kurzkettiger Alkohole finden weitreichende Verwendung. Neben deren eingangsbereits genannten Verwendungsmöglichkeiten sei noch die Bedeutung dieser Ester für die Herstellung von tensidischen Chemikalien oder Vorprodukten wie Alkanolamiden, Zuckerestern oder α-Sulfofettsäureestern erwähnt. Glycerin ist eine wichtige chemische Verbindung, die beispielsweise für die Herstellung von Sprengstoffen, als Zusatz zu Wärme- und Kraftübertragungsflüssigkeiten, als feuchtigkeitserhaltender Zusatz zu Hautcremes, Zahnpasten, Seifen, Tabak und ähnlichem, als Textilhilfsmittel, als Lösungsmittel und auf vielen anderen Gebieten, die dem Fachmann bekannt sind, verwendet werden kann.

Die Erfindung wird durch folgende Beispiele erläutert:

Beispiel 1

In einem beheizbaren Reaktionsgefäß von 800 $cm^3$ Inhalt, versehen mit Rührer, Gaseinleitungsrohr, Innenthermometer und einem kurzen Übergangsaufsatz zu dem aus zwei Kondensationsgefäßen bestehenden Vorlagensystem, in dem die flüchtigen Reaktionsprodukte kondensiert werden, legt man 498 g technischen Talg (Verseifungszahl 195, Säurezahl 1) zusammen mit 27 g an 30 gew.-%igem Natriummethylat (in Methanol) vor. Der Talg wird auf 240 °C erwärmt und bei dieser Temperatur gehalten. Ein Methanolgasstrom von 12,6 mol/h (entsprechend 25,3 mol/1000 g Fett · h) wird in einem Verdampfer kontinuierlich aus flüssigem Methanol erzeugt und durch den flüssigen Talg hindurchgeleitet. Das Kondensationssystem wird bei 90 °C gehalten, so daß das austretende überschüssige Methanolgas danach wieder in den Reaktor zurückgeführt werden kann. Die Reaktion ist nach 3,75 h beendet. Es wird nach dieser Zeit 498,5 g Rohkondensat erhalten, das in einem Absetzgefäß vereinigt wird, wo die Trennung erfolgt. Sodann wird die Glycerinphase abgelassen. Die Fettsäuremethylester-Phase wird zweimal mit je 50 ml Wasser gewaschen, nach Trocknung werden 441 g Talgfettsäuremethylester mit einer Säurezahl (SZ) von 0,8 (das sind 95,8 % der Theorie, korrigiert mit der Säurezahl) erhalten. Das Waschwasser wird mit der Glycerinphase vereinigt und dann das Wasser im Rotationsverdampfer entfernt. Auf diese Weise werden 41,2 g Rohglycerin erhalten. Der Reinglycerin-Gehalt dieses Rohglycerins wird nach der Periodat-Methode titrimetrisch ermittelt. Er beträgt 39,3 g an Glycerin, das sind 69,4 % der Theorie.

Mit der in Beispiel 1 beschriebenen Apparatur sind in diskontinuierlicher Reaktionsweise weitere Versuche durchgeführt worden. Die Ausgangsprodukte, die Menge und Qualität der erhaltenen Endprodukte und die Reaktionsparameter sind in der folgenden Tabelle I zusammengefaßt.

In den Tabellen I und II werden die folgenden Abkürzungen und Bezeichnungen mit der nachstehend wiedergegebenen Bedeutung verwendet:

Glyceride (VZ = Verseifungszahl, SZ = Säurezahl)

A = technischer Talg (VZ 195; SZ 1)
B = Tierkörperfett filtriert (VZ 187; SZ 13; unverseifbare Anteile einschließlich Schleimstoffe 1,2 Gew.-%)
C = Tierkörperfett unfiltriert (VZ 189; SZ 8,6; unverseifbare Anteile einschließlich Schleimstoffe 1,6 Gew.
D = Butterfett roh (VZ 188,5; SZ 1,7; unverseifbare Antei einschließlich Schleimstoffe 1,4 Gew.-%; 12,3 Gew.-% Wasser)
E = technischer Talg (VZ 188,5; SZ 0,7)
F = Kokosöl roh (VZ 244; SZ 1,5)
G = Sojaöl roh (VZ 187,9; SZ 0,4)
H = Rüböl roh (VZ 183,4; SZ 9,4)
K = Rapsöl roh (VZ 183,4; SZ 11,8)
L = Tierkörperfett filtriert (VZ 191,3; SZ 8,5; unverseifbare Anteile einschließlich Schleimstoffe 1,3 Gew.-%
M = Ricinusöl techn. (VZ 176,2; SZ 1,6; OH-Zahl 164,4)
N = Sonnenblumenöl, Speisequalität (VZ 178; SZ 0,1)
O = Olivenöl, Speisequalität (VZ 190; SZ < 0,1 )
P = Palmkernöl roh (VZ 229; SZ 2,8)
R = Glycerintristearat techn. ( VZ 194; SZ 4)
S = Talg techn. ( VZ 190,3; SZ 1,3)
T = Tierkörperfett filtriert ( VZ 182; SZ 9,8)

Alkohole

I = Methanol
II = Ethanol
III = Isopropanol
IV = n-Propanol
V = n-Butanol

Katalysatoren

a = Natriummethylat
b = Zinklaurat
c = Kaliummethylat
d = Cesiumlaurat
e = Zink-Dodecylbenzolsulfonat
f = Kaliumhydroxid
g = Cesiumcarbonat
h = Natriumhydrogencarbonat
P = Temperaturprogramm: 3 Stunden bei 230 °C, Erhöhung um 10 °C/30 min bis 260 °C, bei dieser Temperatur weiter bis Reaktionsende;

* = durchgesetzte Gesamtmenge (bei Aufrechterhaltung einer Füllmenge von 500 g Glycerid im Reaktionsgefäß);
** = Durchsatzmenge in mol/1000 g Glycerid mal Stunde;
+) = Reaktionsgefäß von 400 $cm^3$ Inhalt; Einbauten, Ein- und Auslässe wie beschrieben;
++) = zusätzlich zum Methanolgasstrom werden hier noch 162 Normalliter Stickstoff/1000 g Glycerid mal Stunde das heißt 30 Vol.-%, bezogen auf das Normvolumen, des Methanols hindurchgeleitet.

**TABELLE 1**

| Beisp. Nr. | Glycerid Art | Glycerid Menge (g) | Alkohol Art | Alkohol Durchsatzmenge ** | Katalysator Art | Katalysator Gew.-%, bez. auf Glycerid | Temp. (°C) | Reaktionszeit (h) | Ester-Produkt Menge (g) | Ester-Produkt SZ | Ester-Produkt % der Theorie | Rohglycerin (g) | Glycerin n. Periodat-Methode (g) | % Glycerin d. Theorie |
|---|---|---|---|---|---|---|---|---|---|---|---|---|---|---|
| 2 *) | A | 200,5 | I | 37,5 | a | 0,45 | 230 | 4 | 182,8 | 0,1 | 90,7 | 18,5 | 18,4 | 84,7 |
| 3 | A | 503,0 | I | 22,3 | a | 1,6 | 220 | 7,25 | 448,1 | 0,4 | 96,6 | 41,6 | 39,1 | 71,9 |
| 4 | A | 502,5 | I | 28,4 | a | 1,6 | 270 | 2,25 | 447,0 | 1,2 | 96,4 | 36,5 | 34,1 | 63,2 |
| 5 *) | A | 239,0 | I | 43,7 | b | 1,0 | 250 | 5 | 231,4 | 0,2 | 96,4 | -- | 18,9 | 69,6 |
| 6 *) | B | 200,8 | I | 50,1 | b | 2,0 | 250 | 3 | 183,0 | 0,4 | 91,7 | -- | 13,9 | 66,3 |
| 7 | C | 503,0 | I | 28,8 | c | 2,0 | 240 | 3,25 | 441,7 | 0,8 | 95,7 | 37,9 | 33,8 | 75,0 |
| 8 | A | 501,0 | I | 24,1 **) | d | 0,9 | 240 | 5,75 | 494,2 | 1,2 | 97,6 | 49,7 | 46,5 | 85,8 |
| 9 | A | 496,0 | I | 15,6 | a | 0,2 | P | 7 | 482,0 | 1,2 | 96,2 | 44,9 | 44,4 | 82,7 |
| 10 | D | 486,5 | I | 24,5 | a | 1,6 | 240 | 3 | 329,6 | 0,4 | 85,7 | 37,7 | 35,6 | 74,0 |
| 11 | A | 500,1 | II | 18,1 | a | 1,6 | 240 | 7 | 462,3 | 0,6 | 95,4 | 39,4 | 37,2 | 69,2 |
| 12 | E | 311,0 | III | 21,5 | a | 1,6 | 240 | 20 | 275,2 | 7,2 | 84,4 | 20,0 | 18,2 | 57,0 |
| 13 *) | E | 152,0 | V | 37,4 | a | 1,6 | 240 | 3,75 | 133,7 | 0,2 | 83,3 | 11,7 | 10,4 | 66,8 |
| 14 | F | 418,2 | I | 28,5 | c | 1,1 | 240 | 3 | 391,4 | 3,5 | 95,4 | 44,0 | 42,7 | 83,4 |
| 15 *) | G | 151,5 | I | 39,0 | a | 1,6 | 240 | 3 | 130,2 | 0,1 | 92,7 | 10,6 | 10,5 | 68,2 |

**TABELLE I** (fortgesetzt)

| Beisp. Nr. | Glycerid Art | Glycerid Menge (g) | Alkohol Art | Alkohol Durchsatzmenge ** | Katalysator Art | Katalysator Gew.-%, bez. auf Glycerid | Temp. (°C) | Reaktionszeit (h) | Ester-Produkt Menge (g) | Ester-Produkt SZ | Ester-Produkt % der Theorie | Rohglycerin (g) | Glycerin n. Periodat-Methode (g) | % Glycerin d. Theorie |
|---|---|---|---|---|---|---|---|---|---|---|---|---|---|---|
| 16 | H | 152,0 *) | I | 41,2 | a | 1,6 | 240 | 3 | 139,5 | 0,4 | 98,8 | 11,0 | 10,7 | 74,0 |
| 17 | A | 494,0 | I | 48,0 | e | 0,5 | 240 | 5,75 | 474,3 | 1,6 | 95,6 | 28,4 | 27,0 | 50,5 |
| 18 | S | 495,6 | I | 24,9 | c | 0,1 | 240 | 5,5 | 463,4 | 0,3 | 93,0 | 41,8 | 41,5 | 81,0 |
| 19 | T | 488,8 | I | 19,8 | a | 3,2 | 230 | 7,75 | 381,8 | 0,2 | 94,0 | 24,9 | - | 55,1 |
| 20 | A | 500,0 | I | 10,0 | a | 1,6 | 240 | 9,25 | 434,9 | 0,5 | 94,0 | 41,7 | 40,4 | 74,7 |
| 21 | T | 425,2 | IV | 12,8 | a | 1,6 | 240 | 6,25 | 343,5 | 0,1 | 87,7 | 32,2 | 31,0 | 78,8 |
| 22 | P | 425,3 | I | 31,8 | c | 0,5 | 240 | 2 | 400,8 | 0,2 | 95,2 | 42,5 | 42,4 | 81,0 |
| 23 | M | 403,0 | I | 24,9 | a | 1,6 | 240 | 10,75 | 311,6 | 0,3 | 83,1 | 28,5 | - | 75,8 |
| 24 | N | 485,5 | I | 21,7 | a | 0,5 | 240 | 4,25 | 425,8 | <0,1 | 90,0 | 34,9 | 34,9 | 74,0 |
| 25 | O | 477,5 | I | 28,4 | a | 0,5 | 240 | 4,5 | 458 | 0,1 | 97,8 | 43,2 | 41,2 | 83,1 |
| 26 | R | 401,5 | I | 26,0 | c | 3,2 | 280 | 1,75 | 330 | 0,1 | 88,1 | 20,8 | - | 49,9 |
| 27 | S | 400,0 | I | 31,2 | f | 1,6 | 240 | 3,75 | 358,3 | 0,2 | 97,5 | 33,5 | 33,2 | 81,1 |
| 28 | S | 474,3 | I | 27,9 | g | 1,6 | 240 | 4,75 | 447,6 | 0,1 | 96,6 | 38,9 | 38,8 | 80,0 |
| 29 | S | 406,6 | I | 27,6 | h | 1,6 | 240 | 4 | 371,6 | 0,2 | 96,5 | 34,3 | 33,75 | 80,4 |

Beispiel 30

In der in Beispiel 1 beschriebenen Apparatur wird zusätzlich ein heizbares Dosiergefäß für das in den Reaktor nachzuführende Glycerid installiert. Es werden 500 g technischer Talg (Verseifungszahl 188,5, SZ 0,7) mit 27 g an 30 gew.-%igem Natriummethylat (in Methanol) vorgelegt und, bei 225 °C beginnend, wird gasförmiges Methanol hindurchgeleitet. Die Temperatur wird dann bis auf 240 °C gesteigert, Glycerin und Talgfettsäuremethylester werden ausgetragen. Dabei wird Talg in der Weise nachdosiert, daß im Reaktor eine stets gleiche Menge des Reaktionsgutes vorhanden ist. Bei einer konstanten Temperatur von 240 °C sowie gleichmäßiger Zudosierung von Methanolgas und Fett sowie stetigem Austrag der Reaktionsprodukte wird die Reaktion nach 14,5 h abgebrochen. Insgesamt sind 2008 g Talg (einschließlich der Vorlage von 500 g) und 6350 g Methanol (das entspricht 27,4 mol an Methanol pro 1000 g stationäre Glyceridphase mal Stunde) durchgesetzt worden. Die Kondensationsgefäße werden nach jeweils ca. 3 Stunden in ein Absetzgefäß entleert. Dort wird das Rohkondensat wie in Beispiel 1 aufgearbeitet. Es werden auf diese Weise 1980,3 g getrockneter Talgfettsäuremethylester (SZ 0,4; 98,0 % der Theorie, korrigiert mit der Säurezahl) und 162,1 g Rohglycerin erhalten. Nach der Periodat-Bestimmung sind dies 148,6 g Glycerin (72,2 % der Theorie).

Nach der oben beschriebenen kontinuierlichen Reaktionsweise sind die folgenden weiteren Versuche durchgeführt worden. Die Reaktionsparameter und -Ergebnisse sind in Tabelle II wiedergegeben.

0164643

**TABELLE II**

| Beisp. Nr. | Glycerid Art | Glycerid Menge (g)* | Alkohol Art | Alkohol Durchsatzmenge ** | Katalysator Art | Katalysator Gew.-%, bez. auf Glycerid | Temp. (°C) | Reaktionszeit (h) | Ester-Produkt Menge (g) | Ester-Produkt SZ | Ester-Produkt % der Theorie | Rohglycerin (g) | Glycerin n. Periodat-Methode (g) | % Glycerin d. Theorie |
|---|---|---|---|---|---|---|---|---|---|---|---|---|---|---|
| 30 | A | 1476,4 | I | 21,9 | a | 0,5 | 240 | 13,5 | 1432,9 | 0,4 | 96,4 | 137,0 | 133,0 | 83,7 |
| 31 | E | 2533,2 | I | 31,0 | c | 2,25 | 240 | 17 | 2475,7 | 0,3 | 97,4 | 212,3 | 205,9 | 79,3 |
| 32 | L | 1458,9 | I | 29,5 | c | 2,4 | 240 | 10,25 | 1357,0 | 0,5 | 96,2 | 125,5 | 120,7 | 83,8 |

Patentansprüche

HOE 84/F 904

1. Verfahren zur Herstellung von Fettsäureestern kurzkettiger primärer und sekundärer Alkohole mit 1 bis 5 C-Atomen durch Umesterung von Glyceriden mit solchen kurzkettigen Alkoholen in Gegenwart von Umesterungskatalysatoren bei erhöhten Temperaturen, dadurch gekennzeichnet, daß das flüssige Glycerid bei Temperaturen von mindestens 210 °C mit einem Strom von gasförmigem Alkohol in innigen Kontakt gebracht wird, wobei dieser Strom in seiner Durchsatzmenge, bezogen auf die Zeiteinheit, mindestens so bemessen ist, daß er imstande ist, das gebildete Produktgemisch aus Glycerin und Fettsäureester gemeinsam rasch aus der Reaktionszone auszutragen, worauf das Produktgemisch nach Kondensation einer Phasentrennung in eine Fettsäureester- und in eine Glycerin-Phase unterworfen und der überschüssige gasförmige Alkohol in die Reaktionszone zurückgeführt wird.

2. Verfahren gemäß Anspruch 1, dadurch gekennzeichnet, daß die Durchsatzmenge an gasförmigem Alkohol mindestens 8 mol/kg Glycerid mal Stunde beträgt.

3. Verfahren gemäß einem oder mehreren der Ansprüche 1 bis 2, dadurch gekennzeichnet, daß zu der Durchsatzmenge an Alkohol noch bis zu 30 % Inertgas hinzugefügt werden.

4. Verfahren gemäß einem oder mehreren der Ansprüche 1 bis 3, dadurch gekennzeichnet, daß der Alkohol Ethanol, Propanol, Isopropanol oder Methanol ist.

5. Verfahren gemäß einem oder mehreren der Ansprüche 1 bis 4, dadurch gekennzeichnet, daß der Alkohol Methanol ist.

6. Verfahren nach einem oder mehreren der Ansprüche 1 bis 5, dadurch gekennzeichnet, daß Glycerid im Reaktor vorgelegt und die vorgelegte Menge Glycerid durch Zuführung im Maße des Verbrauchs bei der Umsetzung im wesentlichen aufrechterhalten wird.